# EUROPEAN PATENT APPLICATION

(11) **EP 1 516 623 A1**
(43) Date of publication of application: **23.03.2005**
(21) Application number: 03736266.2
(22) Date of filing: 26.06.2003
(51) Int. Cl.: A61K 31/78, A61P 1/00, A61P 1/10, A23L 1/304

(54) **DRUG FOR PREVENTION AND/OR TREATMENT OF CONSTIPATION AND SYMPTOM CAUSED BY CONSTIPATION**

(30) Priority: 26.06.2002 JP 2002185790; 20.01.2003 JP 2003010905
(71) Applicant: Fuji Yakuhin Co., Ltd., Saitama-shi, Saitama 331-8508 (JP)
(72) Inventor: KATO, Hideo, Katsuyama-shi, Fukui 911-0804 (JP); NAGATA, Osamu, Fukui-shi, Fukui 918-8037 (JP); NAKANO, Hiroyuki, Machida-shi, Tokyo 195-0053 (JP); OKAMOTO, Yasushi, Kuki-shi, Saitama 346-0014 (JP); KURITA, Naoki, Toride-shi, Ibaraki 302-0023 (JP); TANAKA, Ippei, Ageo-shi, Saitama 362-0022 (JP); INOUE, Tadashi, Tachikawa-shi, Tokyo 190-0022 (JP); INOUE, Tsutomu, Funabashi-shi, Chiba 274-0806 (JP); UDA, Jun-ichiro, Saitama-shi, Saitama 331-0047 (JP)
(74) Representative: Sternagel, Fleischer, Godemeyer & Partner
(86) International application number: PCT/JP2003/008124
(87) International publication number: WO 2004/002499

(57) **Abstract**

A medicament for preventive and/or therapeutic treatment of constipation and/or a symptom resulting from constipation which comprises magnesium polycarbophil as an active ingredient. The medicament of the present invention can exhibit high efficacy by promoting a normal defecation without causing a side effect such as a watery stool, an overloose stool, or hypercalcemia.

## Description

### Technical Field

The present invention relates to a pharmaceutical composition comprising magnesium polycarbophil as an active ingredient for preventive and/or therapeutic treatment of constipation and/or a symptom resulting from constipation. Furthermore, the present invention relates to a preparation method of magnesium polycarbophil substantially free from magnesium hydroxide.

### Background Art

Constipation is a disorder with a condition in which stools stay in the intestines for a prolonged period of time and defecation becomes difficult due to absorption of water, and is caused by obstructions of a defecation mechanism by various reasons. The obstructions include functional and organic obstructions. For a treatment of constipation, an alimentary therapy such as an intake of food fiber is important, as well as improvement of a living condition and a stress environment. However, it is not easy to practice these treatments continuously in the contemporary society. Purgatives available in the market as generic drugs exceeds 300 kinds, and their productions tend to increase year by year, which clearly proves the increase of patients who take medicine to improve constipation.

However, actually, many patients of constipation take drugs while they are concerned about a time period of movement after application, a condition of stools, and a side effect, in addition to a habituation and an increasing dose due to continuous applications. From these points of view, drugs available at present are unsatisfactory. Furthermore, for those patients who have difficulty of self control or a control of defecation because of a disease, accident, disorder, advanced age, or other reason, laxatives available at present may make a stool watery or undesirably loose, which may sometimes make clothes and surroundings dirty and cause a problem from viewpoints of hygiene and quality of life. In particular, because patients of constipation who need care with aging are increasing, a defecation control at application of a laxative has become a serious problem for care givers, as well as for patients.

Moreover, in relation to extensive causes of diseases resulting from the diversified society, digestive diseases have become diversified, and as a result, patients with constipation resulting from a functional intestinal disorder, particularly irritable bowel syndrome (hereafter, occasionally abbreviated as "IBS" in the specification) with severe symptoms are increasing, as well as those with conventional constipation. Irritable bowel syndrome is a disease which causes an abnormal defecation resulting from functional abnormality of the lower digestive tract even though no organic disorder exists. The syndrome is generally sub-classified into: a constipation type resulting from delayed transport of contents caused by a spastic contraction and the like in the distal colon; a diarrhea type resulting from promoted transport of contents caused by a peristalsis accentuation and the like; and an alternative type in which constipation and diarrhea are repeated in turn. Abnormal defecation by irritable bowel syndrome is considered to be more frequent in females than in males, and generally, the constipation type is reported to be more frequent in females. For treatment of the constipation type IBS, for example, drugs such as magnesium oxide, cisapride, sodium carmellose, sodium picosulfate and the like, and a combination thereof are used. However, non of these drugs is satisfactory from a viewpoint of regulation of defecation.

Polycarbophil is a resin in which polyacrylic acid is crosslinked by 3,4-dihydroxy-1,5-hexadiene, and the resin is known to have a property of high water retention. As for a polyvalent metal salt of polycarbophil; an improvement of physicochemical properties and water retention property, and an effectiveness for treatment of diarrhea are suggested in the U.S. Patent No.3,297,664. However, the publication contains no disclosure that polycarbophil is effective for constipation.

As for calcium polycarbophil, which is a typical polyvalent metal salt of polycarbophil, pharmaceutical compositions containing said calcium salt as an active ingredient are disclosed in the Japanese Patent Nos. 2625456 and 2609022, Japanese Patent Unexamined Publication (KOKAI) No.(Hei)8-198761, Japanese Patent Publication of International Application (KOHYO) No. (Hei)8-502073, and a pharmaceutical composition comprising the calcium polycarbophil has been approved and distributed in the market as a prescription drug effective for an abnormality of defecation (diarrhea and constipation) in IBS and for digestive symptoms. This pharmaceutical has a high efficacy for diarrhea on the basis of its extremely high water retention. However, the pharmaceutical sometimes fails to yield a satisfactory effect, because it merely retains and uses water in the intestines for treatment of constipation, and as a result, is poor in action of positively loosening stools.

Furthermore, a pharmaceutical composition comprising calcium polycarbophil, calcium ions released from the compound in the intestinal tract are absorbed in the body, which may possibly cause hypercalcemia due to promoted absorption of calcium particularly when used in combination with an activated vitamin D, and an enhancement of cardiac contractility particularly when used in combination with a cardiac glycoside, and further may possibly cause problems of: a decrease of effectiveness by suppression of calcium desorption when used in combination with an anti-acid agent or an anti-ulcer agent which suppresses gastric acid secretion; a precipitation of limes and the like to tissues; a generation of a calculus consisting of insoluble or slightly soluble calcium salts such as calcium oxalate and the like; and an inhibition of absorption of other pharmaceuticals based on a chelate formation by calcium ions. In particular, with an increase of health orientated trend, numbers of foods and non-prescription drugs containing activated vitamin D and a large amount of calcium ion are commercially available, and when the foods or the non-prescription drugs are used in combination with a pharmaceutical comprising calcium polycarbophil, a risk may possibly arise in that a disease such as hypercalcemia or a renal disorder resulting from insoluble or a slightly soluble calcium salts is triggered.

As for preparation of magnesium polycarbophil, Example 30 described in the specification of U.S. Patent No.3,297,664 discloses a method comprising addition of a stoichiometric amount of magnesium oxide to polycarbophil and successive stirring overnight, followed by collection of a product by filtration and washing with water. However, this method has a problem in that magnesium hydroxide, derived from magnesium oxide used as a reagent, is get mixed into magnesium polycarbophil as the target compound. For preparations of pharmaceuticals, elimination of contamination of impurities other than an active ingredient is required as much as possible. The inventors of the present invention conducted various researches to decrease the contamination of magnesium oxide in the aforementioned method by examining various conditions such as an amount of magnesium oxide to be reacted, a way of addition, and a temperature condition, as well as a treatment method such as filtration and washing after the reaction. However, no satisfactory result was obtained. Moreover, this method requires a reaction at a high temperature and for a prolonged period of time, and further raises a problem in that operability such as stirring and filtration was poor.

### Disclosure of the Invention

The first object of the present invention is to provide a medicament for preventive and/or therapeutic treatment of constipation and/or a symptom resulting from constipation. More specifically, the object of the present invention is to provide a medicament for the preventive and/or therapeutic treatment of constipation and/or a symptom resulting from constipation, which is superior in safety and efficacy and successfully exerts a high effectiveness without causing a side effect such as a watery stool, an overloose stool, hypercalcemia and the like.

The second object of the present invention is to provide a method for preparation of magnesium polycarbophil with a high purity which is substantially free from magnesium hydroxide. Further object of the present invention is to provide a method for efficient preparation of magnesium polycarbophil having a high purity.

The inventors of the present invention conducted various researches to achieve the aforementioned first object, and as result, they found magnesium polycarbophil, which is a magnesium salt of polycarbophil, had high effectiveness for preventive and/or therapeutic treatment of constipation and/or symptoms resulting from constipation without causing a side effect such as a watery stool and an overloose stool. The present invention was achieved on the basis of the aforementioned findings.

The present invention thus provides a medicament for preventive and/or therapeutic treatment of constipation and/or a symptom resulting from constipation which comprises magnesium polycarbophil as an active ingredient. According to a preferred embodiments of the present invention, provided are the aforementioned medicament which is used as a laxative; the aforementioned medicament which is used for a preventive and/or therapeutic treatment of constipation and/or a symptom resulting from constipation in a functional intestinal disease; the aforementioned medicament which is used for a preventive and/or therapeutic treatment of constipation and/or a symptom resulting from constipation in an irritable bowel syndrome; the aforementioned medicament which is used for a prevention, a remission, an improvement, or a therapeutic treatment of an abnormal defecation or an abnormal digestive tract resulting from constipation; and the aforementioned medicament which is a pretreatment agent for a bowel examination.

From another aspect, a use of magnesium polycarbophil for manufacture of the aforementioned medicament is provided by the present invention. Furthermore, a method for preventive and/or therapeutic treatment of constipation and/or a symptom resulting from constipation is provided by the present invention which comprises the step of administering a preventively and/or a therapeutically effective amount of magnesium polycarbophil to a mammal including human.

The inventors of the present invention further conducted intensive researches to achieve the second object, and as a result, they found that, by using a magnesium carbonate compound or a magnesium phosphate compound among variety of magnesium compounds, magnesium polycarbophil was conveniently obtained which had an extremely high purity and was substantially free from magnesium hydroxide. The present invention was achieved on the basis of the aforementioned findings.

The present invention thus provides a method for preparation of magnesium polycarbophil which comprises the step of reacting polycarbophil with a magnesium carbonate compound or a magnesium phosphate compound. This method can be used as a method for preparation of magnesium polycarbophil substantially free from magnesium hydroxide. According to a preferred embodiment of the present invention, provided is the aforementioned method wherein the magnesium carbonate compound is magnesium carbonate or magnesium carbonate hydroxide.

From another aspect, magnesium polycarbophil is provided which is substantially free from magnesium hydroxide. Preferably, provided is magnesium polycarbophil substantially free from magnesium hydroxide which is obtainable by the aforementioned method. This magnesium polycarbophil is suitably used as an active ingredient of the aforementioned medicament.

From further aspect, a medicament comprising magnesium polycarbophil which is substantially free from magnesium hydroxide is provided by the present invention. According to preferred embodiments of the invention, provided are a medicament for preventive and/or therapeutic treatment of constipation and/or a symptom resulting from constipation which comprises as an active ingredient magnesium polycarbophil substantially free from magnesium hydroxide; the aforementioned medicament which is used as a laxative; the aforementioned medicament which is used for preventive and/or therapeutic treatment of constipation and/or a symptom resulting from constipation in a functional intestinal disease; the aforementioned medicament which is used for a preventive and/or therapeutic treatment of constipation and/or a symptom resulting from constipation in an irritable bowel syndrome; the aforementioned medicament which is used for a prevention, a remission, an improvement, or a therapeutic treatment of an abnormal defecation or an abnormal digestive tract resulting from constipation; and the aforementioned medicament which is a pretreatment agent for a bowel examination.

A food is also provided by the present invention which contains magnesium polycarbophil substantially free from magnesium hydroxide.

### Brief Explanation of Drawings

Fig. 1 is a powder X-ray diffraction chart of a highly pure magnesium polycarbophil substantially free from magnesium hydroxide obtained by the method of the present invention.

Fig. 2 is a powder X-ray diffraction chart of magnesium polycarbophil prepared by the method described in the specification of the U.S. Patent No.3,297,664 wherein magnesium oxide was used as a reagent. In the figure, the lower part shows diffraction angles of magnesium hydroxide (Mg(OH)₂) and magnesium oxide (MgO).

### Best Mode for Carrying out the Invention

The medicament of the present invention is used for preventive and/or therapeutic treatment of constipation and/or a symptom resulting from constipation, and is characterized to comprise magnesium polycarbophil as an active ingredient. Magnesium polycarbophil used as the active ingredient of the medicament of the present invention is a known substance, and any magnesium polycarbophil prepared by any preparation method can be used. For example, magnesium polycarbophil may be easily prepared by the method described in the specification of the U.S. Patent No. 3,202,577. More specifically, acrylic acid and a hexadiene compound such as 3,4-dihydroxy-1,5-hexadiene is copolymerized by using a reaction initiator to obtain polycarbophil, and then the product is washed with water and isolated as a magnesium salt. A magnesium salt may also be prepared and isolated from commercially available polycarbophil and a magnesium containing substance such as magnesium oxide.

Alternatively, by using methacrylic acid, acrylate, maleic acid anhydrate, or fumaric ester and the like instead of the acrylic acid, and by using divinylbenzene, diisopropenylbenzene, tetraaryl-1,3-propanediol and the like instead of the hexadiene compound, and further by using azobisisobutyronitrile, benzoylperoxide and the like as the reaction initiator, the copolymer can be obtained by a reaction in a similar manner, and then the copolymer is treated with a magnesium containing substance such as magnesium oxide to obtain magnesium polycarbophil having a favorably high water absorption property. Preparations of magnesium polycarbophil are specifically exemplified in Examples of the specification, and accordingly, those skilled in the art can easily obtain magnesium polycarbophil by referring to the aforementioned general explanations about the methods for preparation and specific explanations in Examples, and if necessary, by adding an appropriate modification or alteration to these methods.

As the active ingredient of the medicament of the present invention, it is preferred to use magnesium polycarbophil substantially free from magnesium hydroxide, which is obtainable by reacting polycarbophil with a magnesium carbonate compound or a magnesium phosphate compound.

The term "magnesium carbonate compound" used herein means compounds containing MgCO₃ as a constituent element of a salt, and an arbitrary number of metal ion other than MgCO₃, hydroxide ion, or water molecule may be included as a constituent element of a salt. Examples of the magnesium carbonate compound include, for example, magnesium carbonate anhydride, magnesium carbonate monohydrate, magnesium carbonate trihydrate, magnesium carbonate pentahydrate (there magnesium carbonates may sometimes be classified as hydrated basic magnesium carbonate, hydrated normal magnesium carbonate, magnesium carbonate heavy, magnesium carbonate light and the like), calcium magnesium carbonate, magnesium carbonate hydroxide (which is a basic salt represented approximately as 4MgCO₃ · Mg(OH)₂ · 5H₂O, and is also called magnesium alba or magnesium carbonate. The substance described as "magnesium carbonate" in the 14th edition of the Japanese Pharmacopoeia is the aforementioned magnesium carbonate hydroxide.). Among them, magnesium carbonate anhydride or magnesium carbonate hydroxide is preferably used. Magnesium carbonate hydroxide is most preferred.

The term "magnesium phosphate compound" used herein means compounds containing Mg₃(PO₄)₂ as a constituent element of a salt, and an arbitrary number of metal ion other than Mg₃(PO₄)₂, hydroxide ion, or water molecule may be included as a constituent element of a salt. Examples of the magnesium phosphate compound include, for example, magnesium phosphate anhydride, magnesium phosphate tetrahydrate, magnesium phosphate octahydrate, magnesium phosphate docosahydrate and the like.

Polycarbophil used for the reaction is described, for example, in the U.S. Patent No. 3,202,577 and Japanese Patent Unexamined Publication (KOKAI) No.(Hei)4-227911, and can be prepared by copolymerizing acrylic acid and a 1,5-hexadiene compound with a polymerizing agent in the presence of a reaction initiator. In the above reaction, by using methacrylic acid, acrylate, maleic acid anhydrate, or fumaric ester and the like instead of the acrylic acid, and 3,4-dihydroxy-1,5-hexadiene can be used as the 1,5-hexadiene compound. Further, divinylbenzene, diisopropenylbenzene, tetraaryl-1,3-propanediol and the like can be used instead of the 1,5-hexadiene compound. The reaction initiator can be chosen from those ordinarily used such as azobisisobutyronitrile, benzoylperoxide and the like.

In general, a reaction of polycarbophil with a magnesium carbonate compound or a magnesium phosphate compound can be carried out, in the presence of a dispersion medium such as water, by adding a magnesium carbonate compound or a magnesium phosphate compound to polycarbophil, or adding water to polycarbophil and magnesium carbonate hydroxide and the like. For the above reaction, for example, polycarbophil is dispersed in water, and then a magnesium carbonate compound or a magnesium phosphate compound can be added gradually or as one portion to the resulting dispersion liquid. Alternatively, in the presence of a dispersion medium such as water, a method is employable in which polycarbophil is added to a magnesium carbonate compound or a magnesium phosphate compound. For the latter method, polycarbophil in a state of a dispersion in water may be added to the reaction liquid. As a dispersion medium, ion exchanged water, distilled water for injection, or highly deionized so-called purified water may be used, as well as service water ordinarily available. It is also preferable to use sterilized water.

A reaction temperature in the reaction of polycarbophil with a magnesium carbonate compound or a magnesium phosphate compound is not particularly limited. When polycarbophil is mixed with a magnesium carbonate compound or a magnesium phosphate compound, the reaction may be carried out at a temperature in a range of from room temperature to 100°C, as being a reaction temperature after mixing, and from a viewpoint of operability such as a filtration after the reaction, a temperature of from 30 to 85°C is preferable, and about 50°C is most preferred.

A reaction amount of a magnesium carbonate compound or a magnesium phosphate compound based on polycarbophil is not particularly limited. Generally, 1 mole of the aforementioned magnesium compound is reacted with 2 moles of carboxyl group derived from acrylic acid of polycarbophil. When a problem of residue or removal of the aforementioned magnesium compound after the reaction may not arise, 1 to 1.2 moles of the aforementioned magnesium compound may be used based on 2 moles of carboxyl group derived from acrylic acid of polycarbophil. A reaction time is not particularly limited, and may be, in general, several minutes to 1 day, preferably 1 to 12 hours. By employing the above reaction, magnesium polycarbophil can be obtained as fine particles usually assuming white to slightly pale yellow color.

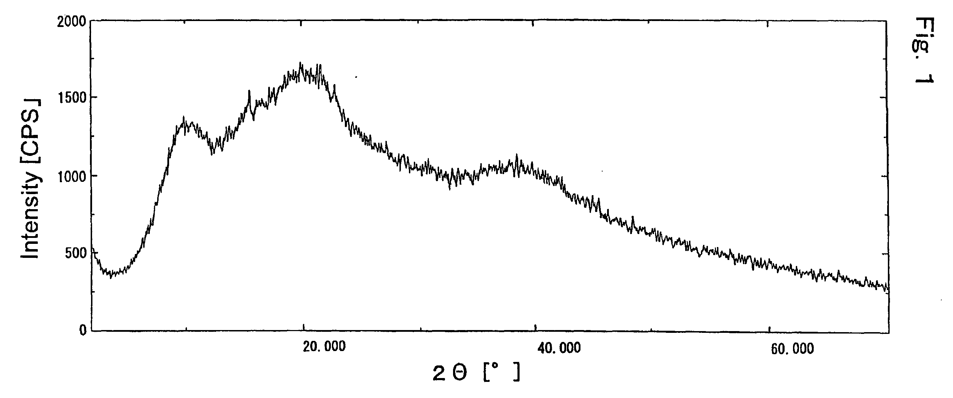

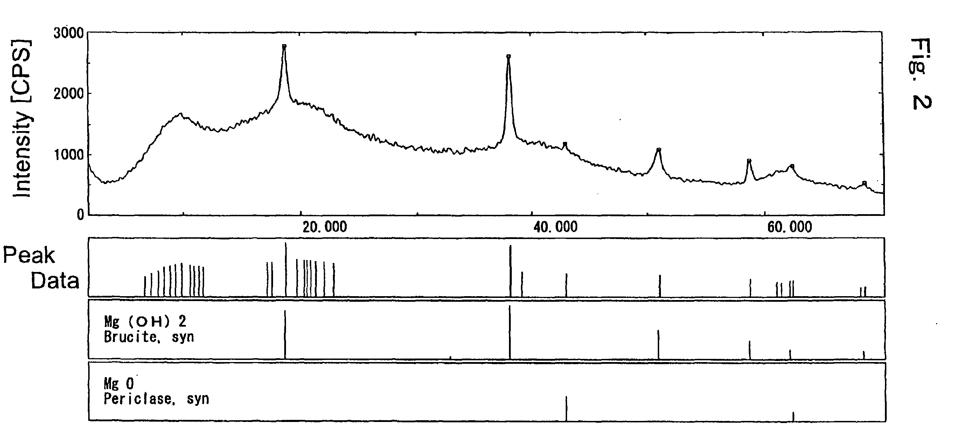

Magnesium polycarbophil obtained by the method of the present invention is characterized to be substantially free from magnesium hydroxide. In the specification, the wording "substantially free from magnesium hydroxide" means, for example, a diffraction peak specific to magnesium hydroxide crystal is not substantially observed in a powder X-ray diffraction chart. More specifically, as shown in Fig. 1, the wording means that any diffraction peak of magnesium hydroxide is not observed in a powder X-ray diffraction chart. For comparison, a powder X-ray diffraction chart of magnesium polycarbophil with a low purity is shown as Fig. 2, which gives a specific diffraction peak of magnesium hydroxide. This magnesium polycarbophil was prepared by the method described in the specification of the U.S. Patent No. 3,297,664.

Furthermore, the magnesium polycarbophil obtained by the method of the present invention contains approximately 13% of magnesium measured by the quantification method of magnesium oxide according to the Japanese Pharmacopoeia, and has a water absorbing capacity of 50 to 80 g/g when measured by the following method. The water absorbing capacity was obtained as a weight of absorbed water corresponding to a swelling in a test solution of 1.5g/dl sodium hydrogen carbonate after a de-magnesium treatment of magnesium polycarbophil with a test solution of 0.1 mol/L hydrochloric acid to afford a free form of polycarbophil.

As the medicament of the present invention, magnesium polycarbophil, per se, may be used, or alternatively, a pharmaceutical composition for an oral administration may be prepared and administered using one or more kinds of pharmaceutical additives widely used in the art. Kinds of the pharmaceutical composition for oral administration are not specifically limited. Examples include solid preparations such as tablets, capsules, granules, subtilized granules, and powders, or aqueous preparations such as suspensions and syrups. Among them, solid preparations such as tablets, capsules, and granules are preferable. For a tablet, an appropriate coating such as an enteric coating may be applied to control solubility and disintegration property, as well as a coating such as a sugar coating or a gelatin coating. Kinds of pharmaceutical additives are not particularly limited. For example, excipients, binders, disintegrators, lubricants, colorants, and corrigents may be used in an appropriate combination. In order to promote disintegration of the pharmaceutical composition, for example, means proposed for pharmaceutical compositions comprising calcium polycarbophil, described in Japanese Patent No.2,609,022, Japanese Patent Unexamined Publication (KOKAI) No.(Hei)8-198761 or the like, may appropriately be used.

More specifically, pharmaceutical compositions may be prepared by using a disintegrator such as sodium carboxymethyl starch, carmellose, croscarmellose sodium, calcium stearate, crystalline cellulose, carmellose sodium, cellulose acetate phthalate, wheat starch, low substituted hydroxypropyl cellulose, methylcellulose, corn starch, potato starch, hydroxyethylmethyl cellulose, hydroxypropyl starch, hydroxypropylmethyl cellulose, magnesium aluminometasillicate, and partially gelatinized starch; a desiccating agent such as ethyl cellulose, magnesium silicate, light anhydrous silicic acid, synthetic aluminum silicate, magnesium stearate, purified shellac, and macrogol; a dispersant such as carboxyvinyl polymer, sucrose fatty acid ester, various polyoxyethylene hydrogenated caster oil, various polysorbate; and a stabilizer such as potassium stearate and aluminum stearate, and further by using a disintegrating aid, a surfactant, a filler, a lubricant and the like, and by appropriately admixing a corrigent, an aromatics and the like, if necessary, and then granules are prepared by granulation, and further, tables can be prepared by compression after the granulation.

Although it is not intended to be bound by any specific theory, magnesium polycarbophil has an action of softening stools modestly and increasing a defecation amount. Therefore, the medicament of the present invention can be used for preventive and/or therapeutic treatment of constipation. Furthermore, the medicament of the present invention can be used for preventive and/or therapeutic treatment of symptoms resulting from constipation such as a digestive symptom. Kinds of constipation applicable by the medicament of the present invention are not particularly limited. The medicament is applicable to any of constipation based on a functional disorder such as a decrease in colic motility, colonospasm, or a decrease of defecation reflex in rectum, or constipation based on an organic disorder such as an enteric transit disorder, a malformation of colon, or a rectum and anal stenosis. Moreover, the medicament is applicable to both of acute constipation and chronic constipation. According to another classification, the medicament is applicable to any of transient simple constipation, habitual (ordinary) constipation, (such as atonic constipation, proctogenic constipation, or spastic constipation), or a symptomatic constipation (a systematic disease such as an organic disorder of colon, hypothyreosis, peptic ulcer, pancreatic disease, biliary tract disease). Furthermore, the medicament is applicable to constipation induced by a drug, for example, by an administration of a narcotic analgesic. Among them, constipation by a functional intestinal disorder is a preferable disease applicable by the medicament of the present invention, and among them, particularly preferred applicable diseases include constipation type irritable bowel syndrome and constipation in alternating irritable bowel syndrome. The medicament of the present invention can be used as a pretreatment drug for an enteric cleaning before a colic examination such as irrigoradioscopy or endoscopy.

Furthermore, although it is not intended to be bound by any specific theory, a positive secretion of water into the intestinal tract in induced by an action of magnesium when polycarbophil is used as a magnesium salt, and as a result, stools may become effectively loose, whilst on the basis of a high water-retaining effect of polycarbophil, a watery stool or an overloose stool can be avoided. Moreover, although it is not intended to be bound by any specific theory, as shown in the examples of the present description, when calcium polycarbophil which is a calcium salt of polycarbophil is used in combination with magnesium oxide usually used as a laxative, diarrhea is caused even at a low dose, whilst the medicament of the present invention does not cause a side effect of diarrhea. The superior action of the medicament of the present invention, by which a high effectiveness to constipation is achieved without causing diarrhea as a side effect, can only be achieved by using magnesium as a counter ion of polycarbophil. Thus, it has been proven that the medicament is qualitatively different from therapeutics in which magnesium polycrbophil is used in combination with a salt-type purgative containing magnesium such as magnesium oxide.

In addition, since an intestinal absorption of magnesium is lower than that of calcium, and also the absorption is not hardly influenced by Vitamin D, it is expected that the medicament of the present invention is expected to lower a risk of inducing hyper-alkali-earth-metalemia. Moreover, since calcium phosphate and calcium oxalate have extremely poor water solubility and often become a major component of a calculus, calcium polycarbophil may have a risk of causing a renal disorder or a calculus by these slightly soluble or insoluble calcium salts. Whilst, it is reported that magnesium increases the solubility of calcium oxalate, thereby inhibits the precipitation of calcium oxalate and calcium phosphate, and thus magnesium hydroxide lowers a recurrence rate of calculus (for example, Journal of Urology Vol.124: 770-774(1980)). A medicament comprising magnesium oxide has been available in Japan which has approved application for "prevention of occurrence of urinary calcium oxalate calculus". Therefore, by using the medicament of the present invention, which comprises a magnesium salt of polycarbophil, it is possible to reduce a risk of causing a renal disorder and a calculus. The medicament of the present invention has twice or more higher effectiveness than calcium polycarbophil in a therapeutic effect of constipation. Accordingly, reduction of a dose may improve the absorption of other pharmaceuticals based on a chelate formation.

Dose and frequencies of administration of the medicament of the present invention are not particularly limited, which may appropriately be chosen depending on degree of symptoms, conditions of a patient such as age, body weight, and sexuality, or a kind of drug simultaneously administered. For example, normally, about 100 to 10,000 mg, preferably about 200 to 6,000mg, more preferably about 500 to 3,000mg per day for an adult may be administered orally once or several times. The medicament of the present invention may be administered in combination with other laxative used for treatment of constipation, or can be used in combination with other medicament having the other efficacy.

The food provided by the present invention contains magnesium polycarbophil together with other inorganic ingredients, amino acids and the like as required, and can be used as a health food. Kinds of the food of the present invention are not particularly limited. For example, an appropriate amount of magnesium polycarbophil may be admixed to a healthy drink, a confectionery, and a processed food. Since magnesium polycarbophil is low toxic without absorption from the digestive tract, the food of the present invention containing the aforementioned magnesium polycarbophil is useful as a health food to control an abnormality of a body where constipation is related directly or indirectly, and to maintain the body healthy.

### Examples

The present invention will be explained more specifically by the Examples. However, the scope of the present invention is not limited to the following Examples. In the following Examples, magnesium polycarbophil is occasionally abbreviated as MgPC and calcium polycarbophil as CaPC.

### Example 1: Preparation of magnesium polycarbophil

Polycarbophil 100 g (Noveon Inc. Noveon polycarbophilAA-1) and magnesium oxide 28 g were added with 6 L of distilled water, and the mixture was stirred at 80°C overnight. The magnesium salt produced was collected by filtration, and washed with distilled water. The resulting resin was powdered after being dried at 80°C for 48 hours, then dried at 80°C for 24 hours under reduced pressure to obtain 100 g of white to slightly pale yellow powder.

### Example 2: Preparation of tablet

Magnesium polycarbophil, lactose, corn starch, and cellulose acetate phthalate (or crystalline cellulose) were mixed in the ratio of ingredients shown in the prescription, and the mixture was granulated by adding an appropriate amount of water, and dried at 60°C for 5 hours. The granules were sized by using a 16-mesh sieve, and the calcium stearate was further mixed in the ratio shown in the prescription to obtain a powder for compression. The powder was divided so as to contain 250 mg of magnesium polycarbophil in each tablet, which was compressed to obtain a tablet.

| Prescription examples 1 | |
|---|---|
| Magnesium polycarbophil | 250 mg |
| Lactose | appropriate amount |
| Cellulose acetate phthalate | 60 mg |
| (Or crystalline cellulose) | |
| Corn starch | 20 mg |
| Calcium stearate | 4 mg |
| | Total 400 mg |

### Test example 1: Comparison of effect on defecation between magnesium polycarbophil and calcium polycarbophil :

Effects of CaPC and MgPC on defecation of normal male dogs were compared for two days together with a control group administered with gelatin capsules. Each test substance, per se, was filled in gelatin capsules, and administered orally at a dose shown in Table 1 about 30 minutes after feeding twice a day for 2 days. 100 ml of water was immediately loaded orally after the administration. After the water load, stools were collected for about 24 hours in a metabolic cage for dogs for 3 days including recovery days, and wet weights of the stools were measured. After the collection, the stools were dried in a dehydrator at 80°C for 12 to 24 hours, and dry weights of the stools were measured and water content ratios of the stools were calculated. During the testing period, the presence of defecation and conditions of stools were observed with passage of time, and examinations were carried out by crossover for several times.

**Table 1**

| Group Number | Treatment | Dose | | | Number / group |
|---|---|---|---|---|---|
| | | mg/kg/day | < mg/kg×number of times * > | Days of Administration | |
| 1 | Control group | 0 | < 0×2 > | 2 days | 3 |
| 2 | MgPC | 1,000 | < 500×2 > | | |
| 3 | | 2,000 | < 1,000×2 > | | |
| 4 | CaPC | 2,000 | < 1,000×2 > | | |

| | | | | | |
|---|---|---|---|---|---|
| *: twice a day administration | | | | | |

In Table 2, the weights of stools, amount of water of stool and the water content ratios of the stools on the first administration day are shown. On the first administration day (0-24 hr), MgPC at 2,000 mg/kg significantly increased the wet weight, the dry weight, and the water content of the stools relative to the control group with no change in the water content ratios of the stools. Furthermore, the weights of stools in the 1,000mg/kg of MgPC administered group and those in the 2,000 mg/kg of CaPC administered group were almost the same, which revealed that the effect of MgPC is twice or more higher than that of CaPC.

**Table 2**

| Group Number | Treatment | Dose (mg/kg/ day) | Weight of stool (0-24hr) | | Amount of water of stool(g) | Water content ratio of stool (%) |
|---|---|---|---|---|---|---|
| | | | Wet weight of stool (g) | Dry weight of stool (g) | | |
| 1 | Control group | 0 | 154±2 | 50±1 | 104±3 | 67±1 |
| 2 | MgPC | 1,000 | 212±23 | 67±9 | 146±14 | 69±1 |
| 3 | | 2,000 | 259±25* | 83±7** | 176±18* | 68±1 |
| 4 | CaPC | 2,000 | 209±16 | 73±2 | 136±15 | 65±2 |

| | | | | | | |
|---|---|---|---|---|---|---|
| Data are shown as average of 3 animals ± standard deviation Amount of water of stool= wet weight of stool - dry weight of stool Water content ratio of stool= amount of water of stool/ wet weight of stool ×100(%) *p<0.05, | | | | | | |
| **p<0.01: significant difference from the control group (Dunnett) | | | | | | |

In Table 3, the weight of stool, amount of water of stool and water content ratio of stool and other during the administration period (2 days) are shown. The wet weight, the dry weight, and the amount of water of stool in the MgPC-administered group were dose dependently and significantly increased during the administration period (0-48 hr) as compared with the control group, and the water content ratio of stool was almost the same as that of the control group, which revealed maintenance of the normal condition of the stool. In the CaPC 2000 mg/kg/day administered group, the wet weight and the dry weight of the stool increased significantly. However, the water content ratio of the stool tended to be decreased compared with that of the control group, and the ratio gave a significant decrease than that of the 2000mg/kg/day of MgPC administered group, which revealed a tendency that the stool became dry and hard.

**Table 3**

| Group Number | Treatment | Dose (mg/k g/day) | Weight of stool (0-48hr) | | Amount of water of stool (g) | Water content ratio of stool (%) |
|---|---|---|---|---|---|---|
| | | | Wet weight of stool (g) | Dry weight of stool (g) | | |
| 1 | Control group | 0× 2 days | 333±21 | 109± 5 | 224±17 | 67±1 |
| 2 | MgPC | 1000× 2 days | 427±15* | 138±10* | 290±6 | 68±1 |
| 3 | | 2000× 2 days | 490±26** | 156± 6** | 335±20* | 68±0 |
| 4 | CaPC | 2000× 2 days | 444±26* | 161± 4** | 283±21 | 64±1# |

| | | | | | | |
|---|---|---|---|---|---|---|
| Data are shown as average of 3 animals ± standard deviation Amount of water of stool = wet weight of stool - dry weight of stool Water content ratio of stool = amount of water of stool/ wet weight of stool ×100(%) *p<0.05, | | | | | | |
| **p<0.01: significant difference from the control group (Dunnett) | | | | | | |
| #p<0.05: significant difference from MgPC 2000mg/kg group (t-test) | | | | | | |

It is clearly understood that MgPC has twice or higher effectiveness than CaPC for treatment of constipation, and has shorter time for exhibition of significant efficacy. Furthermore, it is revealed that MgPC promotes defecation under conditions almost nearer to normal level without a decrease in a water content ratio of a stool, which is observed with CaPC, and at the same time without an increase in water content ratio of a stool.

### Test example 2: Effect on defecation of magnesium polycarbophil alone and a combination of calcium polycarbophil and magnesium oxide (MgO)

Effects of MgPC alone and a combination of CaPC and MgO on defecation on normal male dogs were compared. The test substances were orally administered at doses shown in Table 4 about 30 minutes after feeding twice a day. 100 ml of water was immediately loaded orally after the administration. The doses in the combination were calculated based on the amounts of magnesium (Mg) and polycarbophil(PC) contained in MgPC, and the doses of CaPC and MgO were determined so as to be approximately the same as the prescribed dose of MgPC. After the water load, the accumulated stools were collected for about 1 hour after the administration to 24 hours in a metabolic cage for a dog, and wet weights of the stools were measured. After the measurement, the stools were dried in a dehydrator at 80°C for about 24 hours, and dry weights of the stools were measured to calculate amounts of water and water content ratios of the stools. During the experimental period, the presence of defecation and conditions of the stools were observed with passage of time. The examinations were carried out by crossover for several times.

**Table 4**

| Group Number | Treatment | Dose | | Number / group |
|---|---|---|---|---|
| | | mg/kg/day | < mg/kg×number of times *> | |
| 1 | Control group | 0 | < 0×2> | 4 |
| 2 | MgPC | 500 | < 250×2> | 3 |
| 3 | | 1,000 | < 500×2> | |
| 4 | | 2,000 | <1000×2> | |
| 5 | CaPC+ MgO | 534+114 | < 267×2+57×2> | 3 |
| 6 | | 1,068+228 | < 534×2+114×2> | |
| 7 | | 2,136+454 | <1,068×2+227×2> | |

| | | | | |
|---|---|---|---|---|
| *: twice a day administration "+" in the table indicates combined administration | | | | |

The conditions of the stools are shown in Table 5. Pasty stool and watery stool are judged as diarrhea. Normal stools were observed in the control group and groups administered with 500, 1,000 and 2,000 mg/kg/day MgPC, whilst in the combination ofCaPC+MgO diarrhea was observed in entire range of doses from the low to the high dose.

**Table 5**

| Group Number | Treatment | Dose (mg/kg/day) | Number group / | Condition of stool |
|---|---|---|---|---|
| 1 | Control group | 0 | 4 | normal |
| 2 | MgPC | 500 | 3 | normal |
| 3 | | 1,000 | 3 | normal |
| 4 | | 2,000 | 3 | normal |
| 5 | CaPC+MgO | 534+114 | 3 | diarrhea (1/3) ^{a)} |
| 6 | | 1,068+228 | 3 | diarrhea (2/3) |
| 7 | | 2,136+454 | 3 | diarrhea (3/3) |

| | | | | |
|---|---|---|---|---|
| ^{a)} ( ): number of manifestation / number of use | | | | |

Table 6 shows the weights of the stools, the water content ratios of the stools and others. The wet weight, the dry weight, the amount of water, and the water content ratio of the stools increased dose dependently in the groups administered with MgPC at 500, 1,000 and 2,000 mg/kg/day, and as for the wet weight, the dry weight, and the amount of water, a significant differences were observed at 1,000 mg/kg/day or higher dose as compared to the control group. In the group treated with the combination of CaPC + MgO, the wet weight, the dry weight, the amount of water, and the water content of the stools increased dose dependently. As for the wet weight, the amount of water, and the water content ratio, significant differences were observed in the entire range of doses from the low to the high dose as compared with those of the control group, and as for the dry weight, a significant difference was observed at the medium and higher doses. When MgPC alone and CaPC + MgO combination were compared, significant differences were observed in the wet weight, the amount of water, and the water content ratio of stools at the low doses, and significant differences were observed in the amount of water and the water content ratio at high doses. The dry weights at each dose were almost the same and no significant difference was observed.

**Table 6**

| Group Number | Treatment | Dose (mg/k g/day) | Number / group | Weight of stool | | Amount of water of stool (g) | Water content ratio of stool (%) |
|---|---|---|---|---|---|---|---|
| | | | | Wet weight of stool (g) | Dry weight of stool (g) | | |
| 1 | Control Group | 0 | 4 | 102±16 | 32±5 | 71±12 | 69±1 |
| 2 | MgPC | 500 | 3 | 146± 9 | 42±3 | 104± 5 | 71±1 |
| 3 | | 1,000 | 3 | 169± 3* | 47±1* | 122± 4* | 72±1 |
| 4 | | 2,000 | 3 | 220± 2** | 60±2** | 160± 3** | 73±1 |
| 5 | CaPC +MgO | 534 +114 | 3 | 182± 5**# | 44±1 | 138± 6**# | 76±1**# |
| 6 | | 1,068 +228 | 3 | 216±19** | 52±4** | 165±17** | 76±2** |
| 7 | | 2,136 +454 | 3 | 275±21** | 55±6** | 219±18**^{$} | 80±2**^{$} |

| | | | | | | | |
|---|---|---|---|---|---|---|---|
| Amount of water of stool = wet weight of stool - dry weight of stool Water content ratio of stool = amount of water of stool/ wet weight of stool ×100(%) *p<0.05, | | | | | | | |
| **p<0.01: significant difference from the control group (Dunnett) | | | | | | | |
| #p<0.05: significant difference from the group of MgPC 500mg/kg (t-test) | | | | | | | |
| $p<0.05: significant difference from the group of MgPC 2000mg/kg (t-test) | | | | | | | |

From the results of the above experiments, no difference was observed in the increase of the dry weight of the stool between MgPC alone and CaPC + MgO combination. However, the remarkable increase of the amount of water of the stool in the CaPC + MgO combination group caused diarrhea, whilst in the group administered with MgPC alone, it was shown that a normal defecation is promoted, because the amount of water of the stool was matched to the dry weight of the stool. Accordingly, diarrhea is caused by a combination of a salt-type purgative containing Mg and CaPC, whilst MgPC does not induce diarrhea, which clearly indicates that the latter has a superior effect of promoting defecation which is qualitatively different from the effect of combination of a salt-type purgative containing Mg and CaPC.

### Example 3

In the following examples, "Ultra pure water (Trade Name)" (Seiki Yakuhin Kougyou Inc.) was used as water unless otherwise specifically referred to. FYP powder X-ray analysis in the examples was carried out under the following conditions.
Instrument: RINT2500V (Rigaku Denki Inc.)
X-ray tube : Cu-Ka
X-ray output: 50KV-300A
Scan speed : 4.0°/min
Scan Interval : 0.02°
Scan Range : 2 to 70°
Slit: DS-0.5°, RS-0.15mm, SS-0.5°

Polycarbophil 10 g and magnesium hydrogen carbonate 6.7 g were added with 600 ml of water, and the mixture was stirred at 80 to 85°C for 12 hours, and a supernatant liquid was removed after completion of stirring. 500 ml of water was added to the precipitate and stirred, washed, and the precipitate was collected by suction filtration. The resulting precipitate was dried at 70°C for 12 hours and powdered, and further dried under reduced pressure at 120°C for 4 hours and about 14 g of the target compound was obtained as slightly pale yellow powder. The resulting magnesium polycarbophil was examined by X-ray powder analysis. As a result, no peak of magnesium hydroxide was detected (Fig.1).

### Example 4

Polycarbophil 20 g and magnesium hydrogen carbonate 13.4 g were added with 600 ml of water, and the mixture was stirred at room temperature for 1 hour, and further stirred at 80 to 85°C for 1 hour. After completion of stirring, the supernatant liquid was removed. 500 ml of water was added to the precipitate, stirred and washed, and the precipitate was collected by suction filtration. The precipitate was powdered after drying at 70°C for 12 hours, and further dried under reduced pressure at 120°C for 4 hours to give about 25 g of the target compound as slightly pale yellow powder. The resulting magnesium polycarbophil was examined by X-ray powder analysis. As a result, no peak of magnesium hydroxide was detected.

### Example 5

Polycarbophil 20 g and magnesium hydrogen carbonate 13.4 g were added with 600 ml of water, and the mixture was stirred at room temperature for 1 hour, and further stirred at 80 to 85°C for 3 hours. After completion of stirring, the supernatant liquid was removed. The precipitate was added with 500 ml of water, stirred and washed, and the precipitate was collected by suction filtration. The precipitate was powdered after drying at 70°C for 12 hours, and further dried under reduced pressure at 120°C for 4 hours to give about 25 g of the target compound as slightly pale yellow powder. The resulting magnesium polycarbophil was examined by X-ray powder analysis. As a result, no peak of magnesium hydroxide was detected.

### Example 6

Polycarbophil 20 g and magnesium hydrogen carbonate 13.4 g were added with 600 ml of water, and the mixture was stirred at room temperature for 1 hour, and further stirred at 80 to 85°C for 5 hours. After completion of stirring, the supernatant liquid was removed. The precipitate was added with 500 ml of water stirred and washed, and the precipitate was collected by suction filtration. The precipitate was powdered after drying at 70°C for 12 hours, and further dried under reduced pressure at 120°C for 4 hours to give about 25 g of the target compound as white to slightly pale yellow powder. The resulting magnesium polycarbophil was examined by X-ray powder analysis. As a result, no peak of magnesium hydroxide was detected.

### Example 7

Polycarbophil 10 g and magnesium hydrogen carbonate 6.7 g were added with 600 ml of water, and the mixture was stirred at room temperature for 12 hours. After completion of stirring, the supernatant liquid was removed. The precipitate was added with 500 ml of water, stirred and washed, and the precipitate was collected by suction filtration. The precipitate was treated in the same manner as that of Example 1 to give about 14 g of the target compound as white powder. The resulting magnesium polycarbophil was examined by X-ray powder analysis. As a result, no peak of magnesium hydroxide was detected.

### Example 8

Polycarbophil 10 g was added with 600 ml of water and heated at 80 to 85°C, and the mixture was added with magnesium carbonate hydroxide 6.7g and stirred for 12 hours. After completion of stirring, the supernatant liquid was removed. The precipitate was added with 500 ml of water, stirred and washed, and the precipitate was collected by suction filtration. The precipitate was treated in the same manner as that of Example 1 to give about 14 g of the target compound as slightly pale yellow powder. The resulting magnesium polycarbophil was examined by X-ray powder analysis. As a result, no peak of magnesium hydroxide was detected.

### Example 9

Polycarbophil 20 g and magnesium hydrogen carbonate 13.4 g were added with 600 ml of water, and the mixture was stirred at room temperature for 1 hour, and further stirred at 50°C for 3 hours. After completion of stirring, the supernatant liquid was removed. The precipitate was added with 500 ml of water, stirred and washed, and the precipitate was collected by suction filtration. The precipitate was powdered after drying at 70°C for 12 hours, and further dried at 120°C for 4 hours under reduced pressure to give about 25 g of the target compound as slightly pale yellow powder. The resulting magnesium polycarbophil was examined by X-ray powder analysis. As a result, no peak of magnesium hydroxide was detected.

### Example 10

Polycarbophil 200 g and magnesium hydrogen carbonate 13.4 g were added with 6.0 L of water, and the mixture was stirred at 80 to 85°C for 12 hours. After completion of stirring, the supernatant liquid was removed. The precipitate was added with 2.0 L of water, stirred and washed, and the precipitate was collected by suction filtration. The precipitate was powdered after drying at 70°C for 24 hours, and further dried at 120°C for 4 hours under reduced pressure to give 270 g of the target compound as slightly pale yellow powder. The resulting magnesium polycarbophil was examined by X-ray powder analysis. As a result, no peak of magnesium hydroxide was detected.

### Example 11

Polycarbophil 20 g and magnesium hydrogen carbonate 11.6 g (SIGMA; M-7179, Lot 67H1525) were added with 600 ml of water, and the mixture was stirred at 80 to 85°C for 12 hours. After completion of stirring, the supernatant liquid was removed. The precipitate was added with 400 ml of water, stirred and washed, and the precipitate was collected by suction filtration. The precipitate was powdered after drying at 70°C for 24 hours, and further dried at 120°C for 4 hours under reduced pressure to give 25 g of the target compound as slightly pale yellow powder. The resulting magnesium polycarbophil was examined by X-ray powder analysis. As a result, no peak of magnesium hydroxide was detected.

### Example 12

Polycarbophil 200 g and magnesium hydrogen carbonate 134 g were added with 6.0 L of purified water, and the mixture was stirred at 78 to 80°C for 1 hour and 50 minutes. After completion of stirring, the supernatant liquid was removed. The precipitate was added with 2.0 L of purified water, stirred and washed, and the precipitate was collected by suction filtration. The precipitate was powdered after drying at 70°C for 40 hours, and further dried at 120°C for 4 hours under reduced pressure to give 250 g of the target compound as slightly pale yellow powder. The resulting magnesium polycarbophil was examined by X-ray powder analysis. As a result, no peak of magnesium hydroxide was detected.

### Example 13

Polycarbophil 5.0 g and magnesium phosphate 4.68 g were added with 300 ml of water, and the mixture was stirred at 85°C for 24 hours. After completion of stirring, the supernatant liquid was removed. The precipitate was added with 200 ml of water, stirred and washed, and the precipitate was collected by suction filtration. The collected precipitate was powdered after drying at 70°C for 12 hours, and further dried at 120°C for 4 hours under reduced pressure to give 7.7 g of the target compound as slightly pale yellow powder. The resulting magnesium polycarbophil was examined by X-ray powder analysis. As a result, no peak of magnesium hydroxide was detected.

### Example 14

300 ml of water was added with magnesium hydrogen carbonate 6.7 g, and the mixture was added with polycarbophil 10 g with stirring at room temperature, and further stirred at 50°C for 6 hours. After completion of stirring, the supernatant liquid was removed. The precipitate was added with 200 ml of water, stirred and washed, and the precipitate was collected by suction filtration. The precipitate was treated in the same manner as that of Example 1 to give about 11 g of the target compound as slightly pale yellow powder. The resulting magnesium polycarbophil was examined by X-ray powder analysis. As a result, no peak of magnesium hydroxide was detected.

### Example 15

Polycarbophil 10 g and magnesium hydrogen carbonate 6.7 g were added with 300 ml of ordinary water (ordinary tap water), and the mixture was stirred at room temperature for 1 hour, and further stirred at 50°C for 12 hours. The supernatant liquid was removed after completion of stirring. The precipitate was added with 200 ml of ordinary water, stirred and washed, and the precipitate was collected by suction filtration. The precipitate was powdered after drying at 70°C for 12 hours, and further dried at 120°C for 4 hours under reduced pressure to give about 12 g of the target compound as slightly pale yellow powder. The resulting magnesium polycarbophil was examined by X-ray powder analysis. As a result, no peak of magnesium hydroxide was detected.

### Example 16

Polycarbophil 10 g and magnesium carbonate 5.85 g (ACROS ORGANICS; 41340-5000, Lot A016427201) were added with 600 ml of purified water, and the mixture was stirred at 70 to 75°C for 12 hours. The supernatant liquid was removed after completion of stirring. The precipitate was added with 400 ml of purified water, stirred and washed, and the precipitate was collected by suction filtration. The precipitate was powdered after drying at 70°C for 24 hours, and further dried at 120°C for 4 hours under reduced pressure to give about 9.4 g of the target compound as slightly pale yellow powder. The resulting magnesium polycarbophil was examined by X-ray powder analysis. As a result, no peak of magnesium hydroxide was detected.

### Comparative Example 1

Polycarbophil 100 g and magnesium oxide 28 g were added with 6 L of water, and the mixture was stirred at 85°C for 24 hours, and the supernatant liquid was removed after completion of stirring. The precipitate was added with 2 L of water, stirred and washed, and the precipitate was collected by suction filtration. The precipitate was powdered after drying in the same manner as that of Example 7 to obtain 110 g of the target compound as slightly pale yellow powder. The resulting magnesium polycarbophil was examined by X-ray powder analysis. As a result, a peak of magnesium hydroxide was detected (Fig. 2).

### Comparative Example 2

Polycarbophil 10 g and magnesium hydroxide 4.0 g were added with 600 ml of water, and the mixture was stirred at 85°C for 24 hours. As a result, magnesium hydroxide did not dissolve in the solution, and no target compound was obtained.

### Comparative Example 3

Polycarbophil 10 g and magnesium chloride 6.7g were added with 600 ml of water, and the mixture was stirred at 85°C for 24 hours. As a result, no target compound was obtained.

### Comparative Example 4

Polycarbophil 1 g and magnesium sulfate 0.836 g were added with 600 ml of water, and the mixture was stirred at 85°C for 24 hours. As a result, no target compound was obtained.

### Comparative Example 5

Polycarbophil 10 g and magnesium nitrate 17.8 g were added with 600 ml of water, and the mixture was stirred at 85°C for 24 hours. As a result, the reaction solution became acidic (pH 3.0 to 4.0), and no salt was formed to fail to obtain the target compound.

### Comparative Example 6

Polycarbophil 10 g and magnesium phosphinate 18 g were added with 600 ml of water, and the mixture was stirred at 85°C for 24 hours. As a result, no target compound was obtained.

### Comparative Example 7

Polycarbophil 5.0 g and magnesium hydrogen phosphate 6.07 g were added with 300 ml of water, and the mixture was stirred at 85°C for 24 hours. As a result, no target compound was obtained.

### Comparative Example 8

Polycarbophil 10 g and magnesium acetate 14.9 g were added with 600 ml of water, and the mixture was stirred at 85°C for 24 hours. As a result, no target compound was obtained.

### Comparative Example 9

Polycarbophil 5.0 g and magnesium formate 5.2 g were added with 300 ml of water, and the mixture was stirred at 85°C for 24 hours. As a result, no target compound was obtained.

### Comparative Example 10

Polycarbophil 5.0 g and magnesium lactate 8.93 g were added with 300 ml of water, and the mixture was stirred at 85°C for 24 hours. As a result, no target compound was obtained.

### Comparative Example 11

Polycarbophil 5.0 g and magnesium oxalate 5.16 g were added with 300 ml of water, and the mixture was stirred at 85°C for 24 hours. As a result, no target compound was obtained.

### Comparative Example 12

Polycarbophil 5.0 g and magnesium citrate 7.11 g were added with 300 ml of water, and the mixture was stirred at 85°C for 24 hours. As a result, no target compound was obtained.

### Industrial Applicability

The medicament of the present invention can be used for preventive and/or therapeutic treatment of constipation and/or a symptom resulting from constipation, and can exhibit high efficacy by promoting a normal defecation without causing a side effect such as a watery stool, an overloose stool, or hypercalcemia. Furthermore, according to the method of the present invention, magnesium polycarbophil having a high purity, which is substantially free from magnesium hydroxide, can be conveniently prepared.

## Claims

1. A medicament for preventive and/or therapeutic treatment of constipation and/or a symptom resulting from constipation which comprises magnesium polycarbophil as an active ingredient.

2. The medicament according to claim 1, which is used as a laxative.

3. The medicament according to claim 1 or claim 2, which is used for preventive and/or therapeutic treatment of constipation and/or a symptom resulting from constipation in a functional intestinal disease.

4. The medicament according to any one of claims 1 to 3, which is used for preventive and/or therapeutic treatment of constipation and/or a symptom resulting from constipation in an irritable bowel syndrome.

5. The medicament according to claim 1, which is for prevention, a remission, an improvement, or a treatment of an abnormal defecation or of an abnormality of digestive tract resulting from constipation.

6. A medicament as a pretreatment drug for a bowel examination which comprises magnesium polycarbophil as an active ingredient.

7. A food containing magnesium polycarbophil.

8. A method for preparation of magnesium polycarbophil, which comprises the step of reacting polycarbophil with a magnesium carbonate compound or a magnesium phosphate compound.

9. The method according to claim 8, wherein the magnesium carbonate compound is magnesium carbonate or magnesium carbonate hydroxide.

10. The method according to claim 8 or claim 9, which is for preparation of the magnesium polycarbophil substantially free from magnesium hydroxide.

11. Magnesium polycarbophil substantially free from magnesium hydroxide which is obtainable by the method according to any one of claims 8 to 10.

12. Magnesium polycarbophil substantially free from magnesium hydroxide.

13. A medicament comprising the magnesium polycarbophil according to claim 11 or claim 12.

14. The medicament according to any one of claims 1 to 6, wherein the magnesium polycarbophil is that defined in claim 11 or claim 12.

15. The food according to claim 7, wherein the magnesium polycarbophil is that defined in claim 11 or claim 12.
